(19) Europäisches Patentamt
European Patent Office
Office européen des brevets

(11) Numéro de publication : **0 374 040 B1**

# (12) FASCICULE DE BREVET EUROPEEN

(45) Date de publication du fascicule du brevet : **24.03.93 Bulletin 93/12**

(51) Int. Cl.$^5$ : **C07D 277/82,** A61K 31/425

(21) Numéro de dépôt : **89403458.6**

(22) Date de dépôt : **13.12.89**

## (54) Dérivés de l'imino-2 Benzothiazoline, leurs procédés de préparation et les médicaments les contenant.

Le dossier contient des informations techniques présentées postérieurement au dépôt de la demande et ne figurant pas dans le présent fascicule.

(30) Priorité : **15.12.88 FR 8816546**
**13.07.89 FR 8909480**

(43) Date de publication de la demande : **20.06.90 Bulletin 90/25**

(45) Mention de la délivrance du brevet : **24.03.93 Bulletin 93/12**

(84) Etats contractants désignés : **AT BE CH DE ES FR GB GR IT LI LU NL SE**

(56) Documents cités :
**EP-A- 282 971**
**EP-A- 0 050 551**
**FR-A- 2 357 553**
**Neuroscience Lettres 88 (1988) 195 - 200**

(73) Titulaire : **RHONE-POULENC SANTE**
**20, avenue Raymond Aron**
**F-92160 Antony (FR)**

(72) Inventeur : **Gueremy, Claude**
**3 rue Daumesnil**
**F-78800 Houilles (FR)**
Inventeur : **Jimonet, Patrick**
**13 avenue de Normandie**
**F-78450 Villepreux (FR)**
Inventeur : **Mignani, Serge**
**14 allée Jean-Baptiste Clément**
**F-93190 Livry-Gargan (FR)**

(74) Mandataire : **Savina, Jacques et al**
**RHONE-POULENC SANTE, Service Brevets,**
**20 Avenue Raymond Aron**
**F-92165 Antony Cédex (FR)**

EP 0 374 040 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des dérivés de l'imino-2 benzothiazoline de formule :

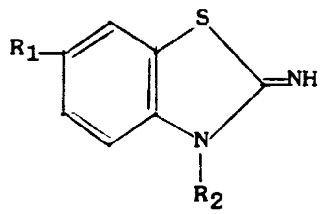

(I)

leurs sels, leurs procédés de préparation et les médicaments les contenant.

Le brevet EP 50551 décrit l'amino-2 trifluorométhoxy-6 benzothiazole comme anxiolytique, anticonvulsivant et hypnotique. Ce produit et certains dérivés d'amino-2 benzothiazole sont également connus comme antagonistes du glutamate (Neuroscience letters 88,195 (1988) et demande de brevet EP 282 971).

Dans la formule (I),

- $R_1$ représente un radical polyfluoroalcoxy ou polyfluoroalkyle et
- $R_2$ représente
  . soit une chaîne $-CH_2-(CH(R_4))n-R_3$ dans laquelle $R_3$ représente un radical dialkylamino, pipéridino, pyrrolidinyl-1, mercapto, acylthio, alkylthio, alkylsulfinyle ou alkylsulfonyle, $R_4$ représente un atome d'hydrogéne ou un radical alkyle et n est égal à 0 ou 1,
  . soit un reste de formule :

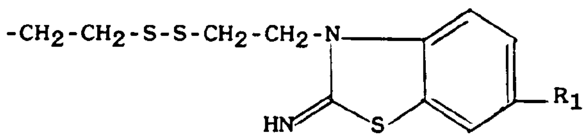

(A)

à l'exception des composés pour lesquels n est égal à 1, $R_3$ représente un radical dialkylamino et $R_4$ représente un radical méthyle.

Sauf mention contraire dans les définitions qui précèdent et celles qui seront citées ci-après, les radicaux alkyle et les portions alkyle et alcoxy contiennent 1 à 4 atomes de carbone en chaîne droite ou ramifiée et les portions acyle contiennent 2 à 4 atomes de carbone.

Les radicaux polyfluoroalcoxy sont de préférence les radicaux trifluorométhoxy, pentafluoroéthoxy, trifluoro-2,2,2 éthoxy ou tétrafluoro-1,1,2,2 éthoxy.

L'invention concerne également les sels d'addition des composés de formule (I) avec les acides minéraux ou organiques.

Les énantiomères des composés de formule (I) comportant un centre asymétrique font également partie de l'invention.

Selon l'invention, les composés de formule (I) à l'exception de ceux pour lesquels $R_2$ représente un reste de formule (A) ou $R_2$ représente une chaîne $-CH_2-(CH(R_4))n-R_3$ dans laquelle $R_3$ représente un radical acylthio peuvent être préparés par action d'un dérivé aminé de formule :

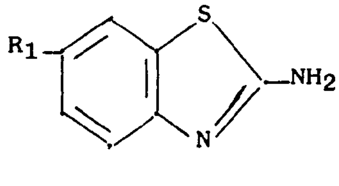

(II)

dans laquelle $R_1$ a les mêmes significations que dans la formule (I) sur un dérivé de formule :

$$R_2 - X \qquad (III)$$

dans laquelle $R_2$ a les mêmes significations que précédemment et X représente un groupe réactif tel qu'un radical tosyloxy ou un atome d'halogène (chlore, brome, iode de préférence) ou un sel d'addition d'un tel composé avec un acide minéral ou organique.

Cette réaction s'effectue généralement dans un solvant organique inerte tel qu'un alcool (éthanol, propanol, par exemple), une cétone (acétone ou méthyléthylcétone par exemple) ou le diméthylformamide, à une température comprise entre 10°C et la température d'ébullition du solvant, éventuellement en présence d'iodure de sodium et éventuellement après fusion à 130-140°C du mélange des composés de formules (II) et (III).

Les composés de formule (II) peuvent être obtenus par application ou adaptation de la méthode décrite par L.M. YAGUPOL'SKII et al, Zh. Obshch. Khim., 33(7), 2301-7, 1963 (Chem. Abst., vol.60, 692 a-f, 1964) ou de la méthode décrite dans le brevet US 2 822 359.

Les composés de formule (III) sont commercialisés ou peuvent être préparés par application ou adaptation de la méthode décrite par T.P. DAWSON, J. Amer. Chem. Soc., 69, 1211 (1947) ou des méthodes décrites dans les exemples.

Selon l'invention, les composés de formule (I) pour lesquels $R_2$ représente une chaîne $-CH_2-(CH(R_4))n-R_3$ dans laquelle $R_3$ représente un radical alkylsulfinyle ou alkylsulfonyle, $R_4$ et n sont définis comme précédemment, peuvent également être obtenus par oxydation des dérivés correspondants pour lesquels $R_3$ représente un radical alkylthio.

L'oxydation en alkylsulfinyle est effectuée généralement au moyen d'acide m-chloroperbenzoïque, au sein d'un alcool, tel que le méthanol ou l'éthanol à une température voisine de -20°C.

L'oxydation en alkylsulfonyle s'effectue généralement au moyen d'eau oxygénée, au sein de l'acide acétique, à une température voisine de 100°C ou au moyen d'acide m-chloroperbenzoïque au sein d'un solvant chloré tel que le chlorure de méthylène ou le chloroforme, à une température voisine de 20°C.

Selon l'invention, les composés de formule (I) pour lesquels $R_2$ représente une chaîne $-CH_2-(CH(R_4))n-R_3$ dans laquelle $R_3$ représente un radical mercapto, $R_4$ et n sont définis comme précédemment, peuvent également être préparés par hydrolyse d'un dérivé correspondant pour lequel $R_3$ représente un radical tertbutylthio.

Cette hydrolyse s'effectue généralement au moyen d'acide bromhydrique, à une température voisine de 110°C.

Les composés de formule (I) pour lesquels $R_2$ représente une chaîne $-CH_2-(CH(R_4))n-R_3$ dans laquelle $R_3$ représente un radical acylthio, peuvent être obtenus par hydrolyse d'un dérivé de formule :

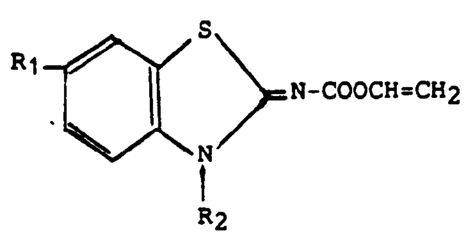

(IV)

dans laquelle $R_1$ a les mêmes significations que dans la formule (I) et $R_2$ a les mêmes significations que précédemment.

Cette réaction s'effectue généralement au moyen d'acide bromhydrique dans l'acide acétique, à une température voisine de 20°C.

Les dérivés de formule (IV) peuvent être obtenus par action d'un dérivé de formule :

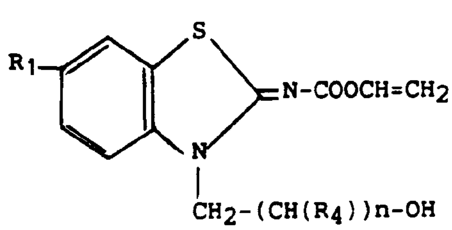

(V)

dans laquelle $R_1$, $R_4$ et n ont les mêmes significations que dans la formule (I) sur l'acide thiolacétique.

Cette réaction s'effectue en présence de triphénylphosphine et d'azodicarboxylate d'éthyle, au sein d'un solvant inerte tel que le tétrahydrofuranne, à une température comprise entre 0 et 20°C.

Les dérivés de formule (V) peuvent être préparés par action d'un dérivé de formule :

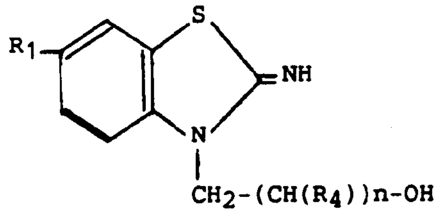

(VI)

dans laquelle $R_1$ a les mêmes significations que dans la formule (I) sur le chlorure de vinyloxycarbonyle.

Cette réaction s'effectue généralement au sein d'un solvant inerte tel que le dichlorométhane, en présence d'une amine tertiaire.

Les dérivés de formule (VI) peuvent être obtenus par action d'un dérivé de formule (II) sur un dérivé de formule (III) dans laquelle $R_2$ représente une chaîne $-CH_2-(CH(R_4))n-OH$.

Cette réaction s'effectue dans les conditions énoncées précédemment pour la réaction des dérivés de formules (II) et (III).

Les composés de formule (I) pour lesquels $R_2$ représente un reste de formule (A) peuvent être préparés par action d'acide bromhydrique sur une (tert-butylthio-2 éthyl)-3 imino-2 polyfluoroalcoxy ou polyfluoroalkyl-6 benzothiazoline.

De préférence, cette réaction s'effectue à une température voisine de 120°C.

Les mélanges réactionnels obtenus par les divers procédés décrits précédemment sont traités suivant des méthodes classiques physiques (évaporation, extraction, distillation, cristallisation, chromatographie ...) ou chimiques (formation de sels ...).

Les énantiomères des composés de formule (I) comportant un centre asymétrique peuvent être obtenus pas dédoublement des racémiques, par exemple, par chromatographie sur colonne chirale selon W.H. PIRKLE et coll., asymetric synthesis, vol. 1, Academic Press (1983) ou encore par synthèse à partir de précurseurs chiraux.

Les composés de formule (I), sous forme de base libre, peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) et leurs sels présentent des propriétés pharmacologiques intéressantes. Ces composés sont actifs vis-à-vis des convulsions induites par le glutamate et sont donc utiles dans le traitement et la prévention des phénomènes convulsifs, des troubles schizophréniques et notamment des formes déficitaires de la schizophrénie, des troubles du sommeil, des phénomènes liés à l'ischémie cérébrale ainsi que des affections neurologiques où le glutamate peut être impliqué telles que la maladie d'Alzheimer, la maladie d'Huntington, la sclérose amyotrophique latérale et l'atrophie olivopontocérébelleuse.

L'activité des composés de formule (I) vis-à-vis des convulsions induites par le glutamate a été déterminée selon une technique inspirée de celle de I.P. LAPIN, J. Neural. Transmission, vol.54, 229-238 (1982) ; l'injection du glutamate par voie intracérébroventriculaire étant effectuée selon une technique inspirée de celle de R. CHERMAT et P. SIMON, J. Pharmacol. (Paris), vol.6, 489-492 (1975). Leur $DE_{50}$ est inférieure ou égale à 3 mg/kg par voie I.P..

Les composés de formule (I) présentent une toxicité faible. Leur $DL_{50}$ est supérieure à 15 mg/kg par voie I.P. chez la souris.

D'un intérêt particulier, sont les composés suivants :

- N,N-diméthylaminoéthyl-3 imino-2 trifluorométhoxy-6 benzothiazoline,
- imino-2 (pipéridino-2 éthyl)-3 trifluorométhoxy-6 benzothiazoline,
- [(pyrrolidinyl-1)-2 éthyl]-3 imino-2 trifluorométhoxy-6 benzothiazoline,
- imino-2 (méthylthio-2 éthyl)-3 trifluorométhoxy-6 benzothiazoline
- (éthylthio-2 éthyl)-3 imino-2 trifluorométhoxy-6 benzothiazoline,
- (éthylsulfinyl-2 éthyl)-3 imino-2 trifluorométhoxy-6 benzothiazoline,
- (éthylsulfonyl-2 éthyl)-3 imino-2 trifluorométhoxy-6 benzothiazoline,
- (imino-2 trifluorométhoxy-6 benzothiazoline-3)-2 éthanethiol,
- imino-2 (méthylsulfinyl-2 éthyl)-3 trifluorométhoxy-6 benzothiazoline,
- (éthylthio-2 éthyl)-3 imino-2 trifluorométhyl-6 benzothiazoline.
- imino-2 méthylthiométhyl-3 trifluorométhoxy-6 benzothiazoline
- imino-2 méthylsulfinylméthyl-3 trifluorométhoxy-6 benzothiazoline
- imino-2 (propylthio-2 éthyl)-3 trifluorométhoxy-6 benzothiazoline
- imino-2 (méthylsulfonyl-2 éthyl)-3 trifluorométhoxy-6 benzothiazoline

- imino-2( éthylsulfinyl-2) éthyl-3 trifluorométhyl-6 benzothiazoline.

Pour l'emploi médicinal, il peut être fait usage des composés de formule (I) tels quels ou à l'état de sels pharmaceutiquement acceptables, c'est-à-dire non toxiques aux doses d'utilisation.

Comme exemples de sels pharmaceutiquement acceptables, peuvent être cités les sels d'addition avec les acides minéraux ou organiques tels que acétate, propionate, succinate, benzoate, fumarate, maléate, oxalate, méthanesulfonate, isothionate, théophyllineacétate, salicylate, phénolphtalinate, méthylène-bis-ß-oxynaphtoate, chlorhydrate, sulfate, nitrate et phosphate.

Les exemples suivants, donnés à titre non limitatif, montrent comment l'invention peut être mise en pratique.

## EXEMPLE 1

7 g d'amino-2 trifluorométhoxy-6 benzothiazole et 4,8 g de chloro-2 N,N-diméthyl éthylamine sont chauffés pendant 1 heure à 130°C. 10 cm3 de propanol-2 sont ajoutés et le chauffage est poursuivi 20 heures à ébullition. Après refroidissement à une température voisine de 20°C, le milieu réactionnel est concentré à 40°C sous pression réduite (20 mm de mercure ; 2,7 kPa) et le résidu traité par 40 cm3 de soude 1N. Après extraction par 100 cm3 de dichlorométhane, séchage sur sulfate de magnésium et concentration à 40°C sous pression réduite, le résidu est purifié par chromatographie sur colonne de silice avec un mélange d'acétate d'éthyle et de méthanol (90-10 en volumes) comme éluant. On obtient 2,1 g de (diméthylamino-2 éthyl)-3 imino-2 trifluorométhoxy-6 benzothiazoline sous forme d'une huile jaunâtre que l'on transforme en dichlorhydrate sublimant vers 200°C.

L'amino-2 trifluorométhoxy-6 benzothiazole peut être préparé selon la méthode décrite par L.M. YAGUPOL'SKII et coll., Zh. Obshch. Khim., 33 (7), 2301 (1963).

## EXEMPLE 2

9,4 g d'amino-2 trifluorométhoxy-6 benzothiazole et 8,1 g de chlorhydrate de N-(chloro-2 éthyl) pipéridine sont chauffés 1 heure à 130°C. On ajoute ensuite 20 cm3 de diméthylformamide et on poursuit la réaction 24 heures à 130°C. Après refroidissement à une température voisine de 20°C, le précipité est filtré, puis traité par 50 cm3 de soude 1N dans 100 cm3 d'eau distillée. Le résidu obtenu par extraction par le dichlorométhane, séchage sur sulfate de magnésium et concentration à sec sous pression réduite (20 mm de mercure ; 2,7 kPa), est purifié par chromatographie sur colonne de silice avec de l'acétate d'éthyle puis un mélange d'acétate d'éthyle et de méthanol (95-5 en volumes) comme éluants. On obtient 2,9 g d'imino-2 (pipéridino-2 éthyl)-3 trifluorométhoxy-6 benzothiazoline sous forme d'une huile jaune que l'on transforme en dichlorhydrate sublimant vers 200°C après recristallisation dans 20 cm3 d'éthanol absolu bouillant.

## EXEMPLE 3

9,4 g d'amino-2 trifluorométhoxy-6 benzothiazole et 7,5 g de chlorhydrate de N-(chloro-2 éthyl) pyrrolidine sont chauffés 2 heures à 130°C. On ajoute ensuite 30 cm3 de propanol-2 et on poursuit la réaction 40 heures à ébullition. Après refroidissement à une température voisine de 20°C, le précipité est filtré, puis traité par 20 cm3 de soude 1N dans 100 cm3 d'eau distillée. Le résidu obtenu par extraction par le dichlorométhane, séchage sur sulfate de magnésium et concentration à sec sous pression réduite (20 mm de mercure ; 2,7 kPa), est repris dans 50 cm3 d'éther éthylique et traité par 3,1 cm3 d'éther chlorhydrique 4,2N . Après recristallisation dans 50 cm3 de propanol-2 bouillant, on obtient 1,9 g de [(pyrrolidinyl-1)-2 éthyl]-3 imino-2 trifluorométhoxy-6 benzothiazoline fondant à une température supérieure à 260°C.

## EXEMPLE 4

Un mélange de 40 g d'amino-2 trifluorométhoxy-6 benzothiazole et de 22,2 g de chloro-1 méthylthio-2 éthane dans 250 cm3 de méthyléthylcétone est chauffé 18 heures à ébullition puis refroidi à une température voisine de 20°C. Le précipité formé est filtré et le filtrat concentré à 40°C sous pression réduite (20 mm de mercure ; 2,7 kPa) jusqu'à un volume d'environ 100 cm3. La réaction est alors poursuivie 72 heures à ébullition puis, après refroidissement à une température voisine de 20°C, le nouveau précipité formé est filtré et réuni au précédent. Après lavage avec 200 cm3 d'éther éthylique, on obtient 31,9 g de chlorhydrate d'imino-2 (méthylthio-2 éthyl)-3 trifluorométhoxy-6 benzothiazoline fondant à 218°C.

EXEMPLE 5

Un mélange de 9,4 g d'amino-2 trifluorométhoxy-6 benzothiazole et de 5,5 g de chloro-1 éthylthio-2 éthane dans 20 cm3 de méthyléthylcétone est chauffé 15 heures à ébullition. Le précipité formé est filtré à chaud, lavé par 2 fois 20 cm3 de méthyléthylcétone bouillant. On obtient 11,5 g de chlorhydrate d'(éthylthio-2 éthyl)-3 imino-2 trifluorométhoxy-6 benzothiazoline sublimant vers 160°C.

EXEMPLE 6

1,5 g de chlorhydrate de (tert-butylthio-2 éthyl)-3 imino-2 trifluorométhoxy-6 benzothiazoline et 15 cm3 d'acide bromhydrique à 47 % sont chauffés 4 heures à 100°C. Après refroidissement à 0°C, le précipité formé est filtré, lavé par 2 fois 25 cm3 d'eau distillée puis par 2 fois 30 cm3 d'éther éthylique. On obtient 0,9 g de bromhydrate d'(imino-2 trifluorométhoxy-6 benzothiazoline-3)-2 éthanethiol fondant à 180°C.

Le chlorhydrate de (tert-butylthio-2 éthyl)-3 imino-2 trifluorométhoxy-6 benzothiazoline peut être préparé selon le procédé suivant : 9,4 g d'amino-2 trifluorométhoxy-6 benzothiazole et 6,7 g de chloro-2 tert-butylthio-1 éthane dans 30 cm3 de méthyléthylcétone sont chauffés 42 heures à ébullition. Après refroidissement du milieu réactionnel à 0°C, le précipité formé est filtré et lavé par 2 fois 20 cm3 de méthyléthylcétone. On obtient 3,7 g de chlorhydrate de (tert-butylthio-2 éthyl)-3 imino-2 trifluorométhoxy-6 benzothiazoline fondant en gommant vers 180-190°C.

Le chloro-2 tert-butylthio-1 éthane peut être préparé selon la méthode décrite par T.P.DAWSON, J.Am. Chem.Soc., 69, 1211 (1947).

EXEMPLE 7

5 g d'(éthylthio-2 éthyl)-3 imino-2 trifluorométhoxy-6 benzothiazoline, 10,3 cm3 d'eau oxygénée à 30 % et 70 cm3 d'acide acétique sont chauffés 24 heures à 100°C. Après refroidissement à une température voisine de 20°C, le milieu réactionnel est ajouté à 200 cm3 d'eau distillée refroidie à 0°C, traité par 120 cm3 de soude à 30 % puis extrait par 300 cm3 d'acétate d'éthyle. La phase organique est lavée avec 150 cm3 d'eau distillée puis par 2 fois 150 cm3 d'une solution aqueuse de bisulfite de sodium, séchée sur sulfate de magnésium et concentrée à sec sous pression réduite (20 mm de mercure ; 2,7 kPa). Après formation du chlorhydrate par addition de 16 cm3 d'acide chlorhydrique 1N dans 25 cm3 de chloroforme, on obtient 1,9 g de chlorhydrate d'(éthylsulfonyl-2 éthyl)-3 imino-2 trifluorométhoxy-6 benzothiazoline fondant à 212°C.

EXEMPLE 8

A 1,7 g d'(éthylthio-2 éthyl)-3 imino-2 trifluorométhoxy-6 benzothiazoline dans 25 cm3 d'éthanol absolu refroidi à -20°C, on ajoute 0,9 g d'acide m-chloroperbenzoïque en environ 10 minutes. La réaction est poursuivie 30 minutes à la même température. Le précipité formé est filtré, puis repris dans 50 cm3 d'éther éthylique et traité par 2 cm3 d'éther chlorhydrique 4N. Après filtration, on obtient 1,1 g de chlorhydrate d'(éthylsulfinyl-2 éthyl)-3 imino-2 trifluorométhoxy-6 benzothiazoline-(RS) fondant à 174°C.

EXEMPLE 9

On opère comme à l'exemple 8, à partir de 3,7 g d'imino-2 (méthylthio-2 éthyl)-3 trifluorométhoxy-6 benzothiazoline et 2,3 g d'acide m-chloroperbenzoïque dans 60 cm3 d'éthanol absolu. Après 30 minutes à -20°C, le précipité formé est filtré et le chlorhydrate préparé dans 60 cm3 d'acétone. On obtient 1,5 g de chlorhydrate d'imino-2 (méthylsulfinyl-2 éthyl)-3 trifluorométhoxy-6 benzothiazoline-(RS) fondant à 186°C.

EXEMPLE 10

6,55 g d'amino-2 trifluorométhyl-6 benzothiazole et 7,48 g de chloro-1 éthylthio-2 éthane dans 20 cm3 de méthyléthylcétone sont chauffés pendant 18 heures à ébullition puis le mélange est refroidi à une température voisine de 20°C. Après concentration à sec à 50°C sous pression réduite (20 mm de mercure ; 2,7 kPa), le résidu est repris dans 50 cm3 de méthyléthylcétone. Le précipité formé est filtré et recristallisé dans 50 cm3 de propanol-2. On obtient 3,1 g de chlorhydrate d'(éthylthio-2 éthyl)-3 imino-2 trifluorométhyl-6 benzothiazoline sublimant vers 160°C.

L'amino-2 trifluorométhyl-6 benzothiazole peut être préparé selon la méthode décrite dans le brevet US 2 822 359.

EXEMPLE 11

16,8g de chlorhydrate de (tert-butylthio-2 éthyl)-3 imino-2 trifluorométhoxy-6 benzothiazoline et 500 cm3 d'acide bromhydrique à 48% sont chauffés à 120°C pendant 18 heures. Le précipité formé est filtré, lavé par 2 fois 50 cm3 d'eau distillée puis par 2 fois 50 cm3 d'éther éthylique. On obtient 10,0g de dibromhydrate de disulfure de bis [imino-2 trifluorométhoxy-6 benzothiazolinyl-3]-2 éthyle dont le point de fusion est supérieur à 260°C.

EXEMPLE 12

Un mélange de 16,4g d'amino-2 trifluorométhoxy-6 benzothiazole et de 8,1g de sulfure de chlorométhyle et de méthyle dans 30 cm3 de méthyléthylcétone est agité 9 heures à une température voisine de 20°C. Le précipité formé est filtré et recristallisé 2 fois dans l'éthanol absolu. On obtient 5,5g de chlorhydrate d'imino-2 méthylthiométhyl-3 trifluorométhoxy-6 benzothiazoline sublimant vers 170°C.

EXEMPLE 13

3,35g de chlorhydrate d'imino-2 méthylthiométhyl-3 trifluorométhoxy-6 benzothiazoline sont ajoutés à 7,6g d'acide m-chloroperbenzoïque en solution dans 70 cm3 de dichlorométhane refroidi à 0°C. La réaction est poursuivie 2 heures à 10-15°C. Le précipité formé est filtré, traité par de la soude aqueuse jusqu'à neutralité et la phase organique extraite par de l'acétate d'éthyle. Après purification par chromatographie sur colonne de silice avec un mélange d'acétate d'éthyle et de méthanol (90-10 en volumes) comme éluant, on obtient 1,0g d'imino-2 méthylsulfinylméthyl-3 trifluorométhoxy-6 benzothiazoline-(RS) transformé sous forme de chlorhydrate sublimant à 150-155°C.

EXEMPLE 14

Un mélange de 9,4 g d'amino-2 trifluorométhoxy-6 benzothiazole et de 6,1 g de chloro-1 propylthio-2 éthane dans 30 cm3 de méthyléthylcétone est chauffé 72 heures à ébullition. Le précipité formé est filtré, lavé par 2 fois 20 cm3 de méthyléthylcétone et recristallisé dans 30 cm3 de propanol-2. On obtient 5,8 g de chlorhydrate d'imino-2 (propylthio-2 éthyl)-3 trifluorométhoxy-6 benzothiazoline fondant à 187°C.

EXEMPLE 15

A 3,4 g d'imino-2 (méthylsulfinyl-2 éthyl)-3 trifluorométhoxy-6 benzothiazoline-(RS) dans 70 cm3 de dichlorométhane refroidi à 0°C, on ajoute 2,1 g d'acide m-chloroperbenzoïque en environ 5 minutes. La réaction est poursuivie 30 minutes à la même température. Le précipité formé est filtré, puis repris dans un mélange de 100 cm3 de dichlorométhane et de 15 cm3 d'éthanol absolu et traité par 6 cm3 d'éther chlorhydrique 4N. Après filtration et recristallisation dans un mélange de 50 cm3 de propanol-2 et de 15 cm3 d'eau distillée, on obtient 2,3 g de chlorhydrate d'imino-2 (méthylsulfonyl-2 éthyl)-3 trifluorométhoxy-6 benzothiazoline sublimant vers 210°C.

EXEMPLE 16

A 3,0 g de chlorhydrate d'(éthylthio-2 éthyl)-3 imino-2 trifluorométhyl-6 benzothiazoline en suspension dans 75 cm3 de dichlorométhane refroidi à 0°C, on ajoute progressivement 6,6 g d'acide m-chloroperbenzoïque. La réaction est poursuivie 2 heures à une température voisine de 20°C. Le précipité formé est filtré puis recristallisé dans 20 cm3 de propanol-2. On obtient 0,8 g de chlorhydrate d'(éthylsulfonyl-2 éthyl)-3 imino-2 trifluorométhyl-6 benzothiazoline sublimant vers 180°C.

EXEMPLE 17

A 5,87g d'imino-2 (propylthio-2 éthyl)-3 trifluorométhoxy-6 benzothiazoline en solution dans 80 cm3 d'éthanol absolu refroidi à -35°C, on ajoute en environ 10 minutes , 4,3g d'acide m-chloroperbenzoïque à 70-75%. La réaction est poursuivie 10 minutes à la même température. Le milieu réactionnel est alors ajouté à 300 cm3 d'éther éthylique et traité par 4,2 cm3 d'éther chlorhydrique 4,2N. Le précipité formé est filtré, puis repris dans 50 cm3 d'eau distillée et neutralisé par de la soude 1N. Après extraction par de l'acétate d'éthyle, séchage sur sulfate de magnésium et concentration à sec sous pression réduite (20 mm de mercure; 2,7kPa),

le produit brut est purifié par chromatographie sur colonne de silice avec de l'acétate d'éthyle puis un mélange d'acétate d'éthyle et de méthanol (90-10 en volumes) comme éluants. On obtient, après transformation en chlorhydrates, 0,33g de chlorhydrate d'imino-2 (propylsulfonyl-2 éthyl)-3 trifluorométhoxy-6 benzothiazoline sublimant vers 200°C et 2,63g de chlorhydrate d'imino-2 (propylsulfinyl-2 éthyl)-3 trifluorométhoxy-6 benzothiazoline-(RS) fondant à 125°C.

EXEMPLE 18

14,2g d'amino-2 trifluorométhoxy-6 benzothiazole et 13,2g bromo-2 méthylthio-1 propane dans 30 cm3 de méthyléthylcétone sont chauffés pendant 3 heures à ébullition. Le précipité formé est filtré, lavé par 2 fois 50 cm3 de méthyléthylcétone et recristallisé dans 50 cm3 de propanol-2. On obtient 11,5g de bromhydrate d'imino-2 (méthylthio-2 propyl)-3 trifluorométhoxy-6 benzothiazoline-(RS) sublimant vers 180°C.

Le bromo-2 méthylthio-1 propane peut être préparé selon la méthode suivante: A 27,6g de méthylthio-1 propanol-2 refroidis à 0°C, on ajoute goutte à goutte 9 ml de tribromure de phosphore. La réaction est poursuivie 4 heures à la même température puis le milieu réactionnel est hydrolysé par addition lente de 50 cm3 d'eau distillée. L'extraction à l'éther éthylique conduit, après séchage, filtration et concentration à sec sous pression réduite (20 mm de mercure; 2,7kPa) à 13,2g de bromo-2 méthylthio-1 propane sous forme d'une huile jaune.

Le méthylthio-1 propanol-2 peut être préparé de la façon suivante: A 17,6g de méthanethiolate de sodium agités à une température voisine de 20°C dans 100 cm3 d'éthanol absolu, on ajoute goutte à goutte 21,3 cm3 de chloro-1 propanol-2. Le milieu réactionnel est alors chauffé à ébullition pendant 3 heures. Après concentration à sec sous pression réduite, le résidu est extrait par 300 cm3 d'acétate d'éthyle, la phase organique lavée à l'eau distillée puis séchée, filtrée et concentrée à sec sous pression réduite. On obtient 19,8g de méthylthio-1 propanol-2 sous forme d'une huile jaune.

EXEMPLE 19

A 6,2g d'imino-2 (méthythio-2 propyl)-3 trifluorométhoxy-6 benzothiazoline-(RS) dans 80 cm3 d'éthanol absolu refroidi à -40°C, on ajoute 4,74g d'acide m-chloroperbenzoïque à 70% en environ 10 minutes. La réaction est poursuivie 10 minutes à la même température. Le milieu réactionnel est ajouté à 200 cm3 d'éther éthylique et traité par 5 cm3 d'éther chlorhydrique 4,2N. Le précipité formé est filtré, puis repris dans 50 cm3 d'eau distillée et traité par de la soude aqueuse jusqu'à neutralité. L'extraction par de l'acétate d'éthyle conduit après traitement habituel à 6,7g de produit brut purifié par chromatographie sur colonne de silice avec un mélange d'acétate d'éthyle et de méthanol (95-5 en volumes) comme éluant. On obtient 0,8g d'imino-2 (méthylsulfonyl-2 propyl)-3 trifluorométhoxy-6 benzothiazoline dont le chlorhydrate sublime vers 200°C, 1,3g d'imino-2 (méthylsulfinyl-2 propyl)-3 trifluorométhoxy-6 benzothiazoline (isomère A) dont le chlorhydrate sublime vers 200°C, et 0,8g d'imino-2 (méthylsulfinyl-2 propyl)-3 trifluorométhoxy-6 benzothiazoline (isomère B) dont le chlorhydrate fond à 134°C.

EXEMPLE 20

3,0g d'(acétylthio-2 éthyl)-3 vinyloxycarbonylimino-2 trifluorométhoxy-6 benzothiazoline dans 30 cm3 d'acide acétique sont agités 18 heures à une température voisine de 20°C en présence de 3 cm3 d'acide bromhydrique à 47%. Après addition de 100 cm3 d'eau distillée et neutralisation à l'aide de soude à 30%, l'extraction par de l'acétate d'éthyle suivie du traitement habituel conduit à 1,2g d'une huile qui cristallise. La formation du chlorhydrate dans un mélange d'éther éthylique et d'acétate d'éthyle par traitement par de l'éther chlorhydrique est suivie d'une recristallisation dans l'acétate d'éthyle conduisant à 0,5g de chlorhydrate d'(acétylthio-2 éthyl)-3 imino-2 trifluorométhoxy-6 benzothiazoline sublimant vers 160°C.

L'(acétylthio-2 éthyl)-3 vinyloxycarbonylimino-2 trifluorométhoxy-6 benzothiazoline peut être obtenu de la manière suivante : A 17,6g de triphénylphosphine en solution dans 150 cm3 de tétrahydrofurane à 0°C, on ajoute goutte à goutte 10,5 cm3 d'azodicarboxylate d'éthyle. L'agitation est poursuivie 30 minutes à cette température. On additionne alors progressivement une solution de 4,8 cm3 d'acide thiolacétique et de 11,7g d'(hydoxy-2 éthyl)-3 vinyloxycarbonylimino-2 trifluorométhoxy-6 benzothiazoline dans 75 cm3 de tétrahydrofurane. La réaction est poursuivie 1 heure à 0°C, puis 1 heure à une température voisine de 20°C. Après concentration à sec sous pression réduite (20 mm de mercure; 2,7kPa), le résidu obtenu est repris dans 50 cm3 de méthanol puis filtré et lavé 1 fois par 50 cm3 de méthanol. On obtient 10,4g d'(acétylthio-2 éthyl)-3 vinyloxycarbonylimino-2 trifluorométhoxy-6 benzothiazoline fondant à 173°C.

L'(hydroxy-2 éthyl)-3 vinyloxycarbonylimino-2 trifluorométhoxy-6 benzothiazoline peut être préparé de la façon suivante : A 20g d'(hydroxy-2 éthyl)-3 imino-2 trifluorométhoxy-6 benzothiazoline en solution dans 200

cm3 de dichlorométhane en présence de 7,9 cm3 de triéthylamine, on ajoute goutte à goutte à 0°C, 4,8 cm3 de chlorure de vinyloxycarbonyle. L'agitation est poursuivie 2 heures à une température voisine de 20°C. Le précipité formé est filtré, lavé à l'eau et séché. On obtient 11,7g d'(hydroxy-2 éthyl)-3 vinyloxycarbonylimino-2 trifluorométhoxy-6 benzothiazoline fondant à 190°C.

L'(hydroxy-2 éthyl)-3 imino-2 trifluorométhoxy-6 benzothiazoline peut être préparé de la manière suivante : 9,4 g d'amino-2 trifluorométhoxy-6 benzothiazole et 10 g de bromo-2 éthanol dans 30 cm3 d'éthanol absolu sont chauffés pendant 95 heures à ébullition. Le mélange est refroidi à une température voisine de 20°C. Le précipité formé est filtré et lavé avec 100 cm3 d'éther éthylique. On obtient ainsi 6,4 g de bromhydrate d'(hydroxy-2 éthyl)-3 imino-2 trifluorométhoxy-6 benzothiazoline fondant à 219°C.

EXEMPLE 21

A une solution refroidie à -5°C de 0,4 g d'(éthylthio-2 éthyl)-3 imino-2 pentafluoroéthoxy-6 benzothiazoline dans 6 cm3 de dichlorométhane, on ajoute en 15 minutes sous agitation une solution séchée sur sulfate de magnésium et préparée à partir de 0,75 g d'acide m-chloroperbenzoïque à 50 % et de 5 cm3 de dichlorométhane. On laisse l'agitation pendant une heure en maintenant la température entre -5°C et 0°C, puis on élimine par filtration l'acide benzoïque formé ; le filtrat est ensuite dilué avec 50 cm3 d'éther éthylique et acidifié avec une solution éthérée d'acide chlorhydrique 5N. On obtient ainsi 0,25 g de chlorhydrate d'(éthylsulfonyl-2 éthyl)-3 imino-2 pentafluoroéthoxy-6 benzothiazoline fondant à 230°C.

L'(éthylthio-2 éthyl)-3 imino-2 pentafluoroéthoxy-6 benzothiazoline peut être préparée de la façon suivante : on chauffe au bain d'huile à 110°C pendant 25 heures un mélange de 0,5 g de pentafluoroéthoxy-6 benzothiazolamine-2, 15 cm3 de méthyléthylcétone et 0,45 cm3 de sulfure de chloro-2 éthyle et d'éthyle. La méthyléthylcétone est évaporée à 45°C sous pression réduite (20 mm de mercure ; 2,7 kPa) et le résidu d'évaporation est repris avec 30 cm3 d'eau, alcalinisé avec de l'ammoniaque à 28 % et extrait trois fois avec 60 cm3 d'éther éthylique au total. Après évaporation sous vide (20 mm de mercure ; 2,7 kPa), le résidu rouge foncé (0,7 g) est purifié par chromatographie sur colonne de silice en éluant avec un mélange de cyclohexane et d'acétate d'éthyle (70-30 en volumes). On obtient ainsi 0,42 g d'(éthylthio-2 éthyl)-3 imino-2 pentafluoroéthoxy-6 benzothiazoline sous forme d'huile qui cristallise avec un point de fusion inférieur à 50°C.

La pentafluoroéthoxy-6 benzothiazolamine-2 peut être préparée de la manière suivante : A une solution de 4,8 g de pentafluoroéthoxy-4 aniline dans 35 cm3 d'acide acétique on ajoute, sous balayage d'argon, 8,15 g de thiocyanate de potassium et on agite pendant 10 minutes à une température voisine de 20°C. A la solution ainsi obtenue, on verse goutte à goutte, en 35 minutes, une solution de 1,1 cm3 de brome dans 10 cm3 d'acide acétique à une température comprise entre 22 et 42°C ; on agite ensuite pendant 20 heures à une température voisine de 20°C. Le mélange réactionnel est versé sur un mélange d'eau et de glace (250 cm3), alcalinisé avec 50 cm3 d'ammoniaque à 28 % et extrait 2 fois avec 250 cm3 d'acétate d'éthyle au total. Après décantation, la solution organique est lavée à l'eau distillée jusqu'à pH 8, séchée sur sulfate de magnésium, filtrée et évaporée à 50°C sous pression réduite (20 mm de mercure ; 2,7 kPa). Le produit obtenu (6,3 g) est purifié par chromatographie sur colonne de silice (650 g, granulométrie : 0,063-0,200 mm) avec comme éluant un mélange de cyclohexane et d'acétate d'éthyle (50-50 en volumes) et recristallisé dans 400 cm3 de cyclohexane bouillant. On obtient 3,25 g de pentafluoroéthoxy- 6 benzothiazolamine-2 fondant à 156°C.

La pentafluoroéthoxy-4 aniline peut être préparée selon la méthode décrite par W.A. SHEPPARD, J. Org. Chem., 29, 1 (1964).

EXEMPLE 22

On opère comme à l'exemple 21, à partir de 6,6 g d'(éthylthio-2 éthyl)-3 imino-2 pentafluoroéthyl-6 benzothiazoline, 12 g d'acide métachloroperbenzoïque à 80 % et 200 cm3 de dichlorométhane. Après chromatographie sur colonne de silice avec l'acétate d'éthyle comme éluant, on récupère 2,6 g d'un solide rose qu'on recristallise deux fois dans un mélange de cyclohexane et d'acétate d'éthyle (80-20 en volumes). On obient ainsi 1 g d'(éthylsulfonyl-2 éthyl)-3 imino-2 pentafluoroéthyl-6 benzothiazoline fondant à 125°C.

L'(éthylthio-2 éthyl)-3 imino-2 pentafluoroéthyl-6 benzothiazoline peut être préparée de la manière suivante : on chauffe à reflux pendant 18 heures un mélange de 11,2 g de pentafluoroéthyl-6 benzothiazolamine-2,6 cm3 de sulfure de chloro-2 éthyle et d'éthyle et 20 cm3 de méthyléthylcétone. Le solvant est ensuite évaporé sous pression réduite (20 mm de mercure ; 2,7 kPa) et le résidu d'évaporation est repris avec 200 cm3 d'eau, alcalinisé avec de l'ammoniaque à 28 % et extrait avec 500 cm3 d'acétate d'éthyle au total. Après évaporation sous vide (20 mm de mercure ; 2,7 kPa), le résidu orangé est purifié par chromatographie sur colonne de silice en éluant avec un mélange de cyclohexane et d'acétate d'éthyle (60-40 en volumes). On obtient ainsi 7,5 g d'(éthylthio-2 éthyl)-3 imino-2 pentafluoroéthyl-6 benzothiazoline sous forme d'huile qui cristallise avec

un point de fusion inférieur à 50°C.

La pentafluoroéthyl-6 benzothiazolamine-2 peut être préparée en opérant comme à l'exemple 21 pour la préparation de la pentafluoroéthoxy-6 benzothiazolamine-2 mais à partir de 14,6 g de pentafluoroéthyl-4 aniline, 14 g de thiocyanate de potassium et 3,6 cm3 de brome dans 150 cm3 d'acide acétique. Après purification sur colonne de silice avec comme éluant un mélange de cyclohexane et d'acétate d'éthyle (50-50 en volumes), on obtient 17 g de pentafluoroéthyl-6 benzothiazolamine-2 fondant vers 100°C.

La pentafluoroéthyl-4 aniline peut être préparée selon la méthode décrite dans le brevet DE2 606982.

EXEMPLE 23

A une solution refroidie à -5°C de 30,8 g d'imino-2 (éthylthio-2) éthyl-3 trifluorométhyl-6 benzothiazoline dans 300 $cm^3$ de dichlorométhane, on ajoute goutte à goutte sous agitation une solution séchée sur sulfate de magnésium et préparée à partir de 38 g d'acide m-chloroperbenzoïque à 50 % et de 300 $cm^3$ de dichlorométhane. On laisse revenir la température à 20°C et on ajoute 100 $cm^3$ d'eau au mélange réactionnel qu'on alcalinise ensuite à pH 10 avec de l'ammoniaque à 28 % ; un précipité blanc apparaît que l'on écarte par filtration. Le flitrat est décanté et la phase organique est séchée sur sulfate de magnésium et évaporée sous pression réduite (20 mm de mercure ; 2,7 kPa). Le résidu d'évaporation est chromatographié sur colonne de silice en éluant avec un mélange d'acétate d'éthyle et d'éthanol (80-20 en volumes). Le produit (26 g) est transformé en chlorhydrate au sein de l'éther éthylique. Après trois recristallisations dans l'éthanol, on obtient 2,9 g de chlorhydrate d'imino-2 (éthylsulfinyl-2) éthyl-3 trifluorométhyl-6 benzothiazo- line-(RS) fondant à 150°C.

L'imino-2 (éthylthio-2) éthyl-3 trifluorométhyl-6 benzothiazoline peut être préparée de la façon suivante : on chauffe à reflux pendant 16 heures un mélange de 32,3 g de trifluorométhyl-6 benzothiazoline-2, 18 $cm^3$ de sulfure de chloro-2 éthyle et d'éthyle et 20 $cm^3$ de méthyléthycétone. Après refroidissement à 20°C, on ajoute 20 $cm^3$ d'acétone et on verse la solution obtenue dans un mélange de 200 $cm^3$ d'acétate d'éthyle et 200 $cm^3$ d'éther de pétrole (40-60°C). Le précipité est filtré, repris avec de l'eau et alcalinisé avec de l'ammoniaque à 28 %. On extrait ensuite la base avec un mélange de 150 $cm^3$ d'acétate d'éthyle et de 150 $cm^3$ de cyclohexane et on la purifie sur colonne de silice (600 g) en éluant avec un mélange d'acétate d'éthyle et de cyclohexane (50-50 en volumes). On obtient 33,2 g d'imino-2 (éthylthio-2) éthyl-3 trifluorométhyl-6 benzothiazoline fondant à 50°C.

La trifluorométhyl-6 benzothiazoline-2 peut être préparée selon le brevet US 2,832,359.

La présente invention concerne également les médicaments constitués par au moins un composé de formule (I) ou un sel d'un tel composé à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, pilules, poudres (capsules de gélatine, cachets) ou granulés. Dans ces compositions, le principe actif selon l'invention est mélangé à un ou plusieurs diluants inertes, tels que amidon, cellulose, saccharose, lactose ou silice.

Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, pommades, lotions,

collyres, collutoires, gouttes nasales ou aérosols.

En thérapeutique humaine, les composés selon l'invention sont particulièrement utiles dans le traitement et la prévention des phénomènes convulsifs, des troubles schizophréniques et notamment des formes déficitaires de la schizophrénie, des troubles du sommeil, des phénomènes liés à l'ischémie cérébrale et des affections neurologiques où le glutamate peut être impliqué telles que la maladie d'Alzheimer, la maladie d'Huntington, la sclérose amyotrophique latérale et l'atrophie olivopontocérébelleuse.

Les doses dépendent de l'effet recherché, de la durée du traitement et de la voie d'administration utilisée ; elles sont généralement comprises entre 30 et 300 mg par jour par voie orale pour un adulte avec des doses unitaires allant de 10 à 100 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

EXEMPLE A

On prépare, selon la technique habituelle, des gélules dosées à 50 mg de produit actif ayant la composition suivante :

| | |
|---|---|
| - (éthylsulfinyl-2 éthyl)-3 imino-2 trifluorométhoxy-6 benzothiazoline | 50 mg |
| - cellulose | 18 mg |
| - lactose | 55 mg |
| - silice colloïdale | 1 mg |
| - carboxyméthylamidon sodique | 10 mg |
| - talc | 10 mg |
| - stéarate de magnésium | 1 mg |

EXEMPLE B

On prépare, selon la technique habituelle, des comprimés dosés à 50 mg de produit actif ayant la composition suivante :

| | |
|---|---|
| - imino-2 (méthylthio-2 éthyl)-3 trifluorométhoxy-6 benzothiazoline | 50 mg |
| - lactose | 104 mg |
| - cellulose | 40 mg |
| - polyvidone | 10 mg |
| - carboxyméthylamidon sodique | 22 mg |
| - talc | 10 mg |
| - stéarate de magnésium | 2 mg |
| - silice colloïdale | 2 mg |
| - mélange d'hydroxyméthylcellulose, glycérine, oxyde de titane (72-3,5-24,5) q. s. p. | 1 comprimé pelliculé terminé à 245 mg |

EXEMPLE C

On prépare une solution injectable contenant 10 mg de produit actif ayant la composition suivante :

| | |
|---|---|
| - (éthylsulfonyl-2 éthyl)-3 imino-2 trifluorométhoxy-6 benzothiazoline | 10 mg |
| - acide benzoïque | 80 mg |
| - alcool benzylique | 0,06 cm3 |
| - benzoate de sodium | 80 mg |
| - éthanol à 95 % | 0,4 cm3 |
| - hydroxyde de sodium | 24 mg |
| - propylène glycol | 1,6 cm3 |
| - eau q. s. p. | 4 cm3 |

**Revendications**

**Revendications pour les Etats contractants suivants : AT, BE, CH, LI, DE, FR, GB, IT, LU, NL, SE**

**1.** Composés de formule :

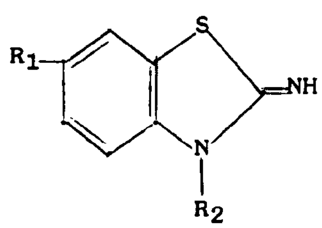
(I)

dans laquelle
- $R_1$ représente un radical polyfluoroalcoxy ou polyfluoroalkyle et
- $R_2$ représente
  . soit une chaîne $-CH_2-(CH(R_4))n-R_3$ dans laquelle $R_3$ représente un radical dialkylamino, pipéridino, pyrrolidinyl-1, mercapto, acylthio, alkylthio, alkylsulfinyle ou alkylsulfonyle, $R_4$ représente un atome d'hydrogéne ou un radical alkyle et n est égal à 0 ou 1,
  . soit un reste de formule :

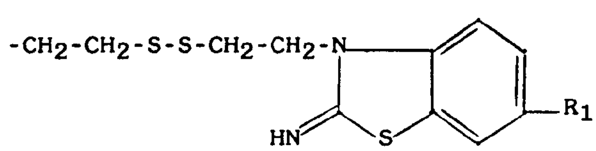
(A)

à l'exception des composés pour lesquels n est égal à 1, $R_3$ représente un radical dialkylamino et R4 représente un radical méthyle, étant entendu que les radicaux alkyle et les portions alkyle et alcoxy contiennent 1 à 4 atomes de carbone en chaîne droite ou ramifiée et les portions acyle contiennent 2 à 4 atomes de carbone, ainsi que les énantiomères des composés comportant un centre asymétrique et les sels de ces composés avec un acide minéral ou organique.

**2.** Procédé de préparation des composés de formule (I) selon la revendication 1 à l'exception de ceux pour lesquels $R_2$ représente un reste de formule (A) ou $R_2$ représente une chaîne $-CH_2-(CH(R_4))n-R_3$ dans la-

quelle $R_3$ représente un radical acylthio caractérisé en ce que l'on fait réagir un dérivé aminé de formule :

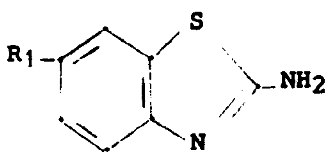

(II)

dans laquelle $R_1$ a les mêmes significations que dans la formule (I) sur un dérivé de formule :

$R_2$ - X (III)

dans laquelle $R_2$ a les mêmes significations que précédemment et X représente un groupe réactif ou un sel d'addition d'un tel composé avec un acide minéral ou organique, isole le produit et le transforme éventuellement en sel d'addition avec un acide minéral ou organique.

3. Procédé de préparation des composés de formule (I) selon la revendication 1 pour lesquels $R_2$ représente une chaîne $-CH_2-(CH(R_4))n-R_3$ dans laquelle $R_3$ représente un radical alkylsulfonyle ou alkylsulfinyle, $R_4$ représente un atome d'hydrogène ou un radical alkyle, n est égal à 0 ou 1 et $R_1$ a les mêmes significations que dans la revendication 1 caractérisé en ce que l'on oxyde les dérivés correspondants pour lesquels $R_3$ représente un radical alkylthio, isole le produit et le transforme éventuellement en sel d'addition avec un acide minéral ou organique.

4. Procédé de préparation des composés de formule (I) selon la revendication 1 pour lesquels $R_2$ représente une chaîne $-CH_2-(CH(R_4))n-R_3$ dans laquelle $R_3$ représente un radical mercapto, $R_4$ représente un atome d'hydrogène ou un radical alkyle, n est égal à 0 ou 1 et $R_1$ a les mêmes significations que dans la revendication 1 caractérisé en ce que l'on hydrolyse un dérivé correspondant pour lequel $R_3$ représente un radical tert-butylthio, isole le produit et le transforme éventuellement en sel d'addition avec un acide minéral ou organique.

5. Procédé de préparation des composés de formule (I) selon la revendication 1 pour lesquels $R_2$ représente un reste de formule (A) et $R_1$ a les mêmes significations que dans la revendication 1 caractérisé en ce que l'on fait réagir de l'acide bromhydrique sur une (tert-butylthio-2 éthyl)-3 imino-2 polyfluoroalcoxy ou polyfluoroalkyle-6 benzothiazoline, isole le produit et le transforme éventuellement en sel d'addition avec un acide minéral ou organique.

6. Procédé de préparation des composés de formule (I) selon la revendication 1 pour lesquels $R_2$ représente une chaîne $-CH_2-(CH(R_4))n-R_3$ dans laquelle $R_3$ représente un radical acylthio caractérisé en ce que l'on hydrolyse un dérivé de formule :

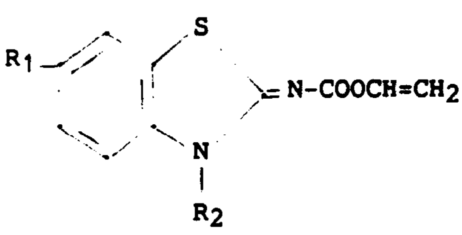

(IV)

dans laquelle $R_1$ a les mêmes significations que dans la revendication 1 et $R_2$ a les mêmes significations que ci-dessus, isole le produit et le transforme éventuellement en sel d'addition avec un acide minéral ou organique.

7. Médicaments caractérisés en ce qu'ils contiennent comme principe actif au moins un composé selon la revendication 1 ou un sel d'un tel composé.

8. Médicaments selon la revendication 6 pour le traitement des affections où le glutamate est impliqué.

9. Composé selon la revendication 1 : (Ethylsulfonyl-2 éthyl)-3 imino-2 trifluorométhoxy-6 benzothiazoline.

**10.** Composé selon la revendication 1 : Imino-2 (méthylsulfinyl-2 éthyl)-3 trifluorométhoxy-6 benzothiazoline.

**Revendications pour les Etats contractants suivants : ES, GR**

**1.** Procédé de préparation des composés de formule

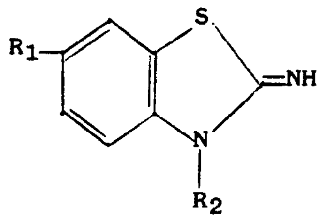

(I)

dans laquelle

- $R_1$ représente un radical polyfluoroalcoxy ou polyfluoroalkyle et
- $R_2$ représente
  . soit une chaîne $-CH_2-(CH(R_4))n-R_3$ dans laquelle $R_3$ représente un radical dialkylamino, pipéridino, pyrrolidinyl-1, mercapto, acylthio, alkylthio, alkylsulfinyle ou alkylsulfonyle, $R_4$ représente un atome d'hydrogéne ou un radical alkyle et n est égal à 0 ou 1,
  . soit un reste de formule :

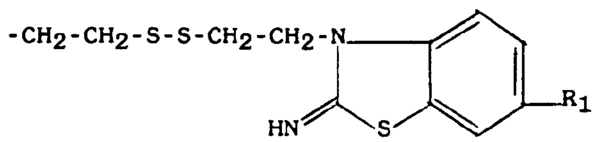

(A)

à l'exception des composés pour lesquels n est égal à 1, $R_3$ représente un radical dialkylamino et $R_4$ représente un radical méthyle, étant entendu que les radicaux alkyle et les portions alkyle et alcoxy contiennent 1 à 4 atomes de carbone en chaîne droite ou ramifiée et les portions acyle contiennent 2 à 4 atomes de carbone, ainsi que les énantiomères des composés comportant un centre asymétrique et les sels de ces composés avec un acide minéral ou organique,

caractérisé en ce que :

A - pour la préparation des composés de formule (I) à l'exception de ceux pour lesquels $R_2$ représente un reste de formule (A) ou $R_2$ représente une chaîne $-CH_2-(CH(R_4))n-R_3$ dans laquelle $R_3$ représente un radical acylthio, on fait réagir un dérivé aminé de formule :

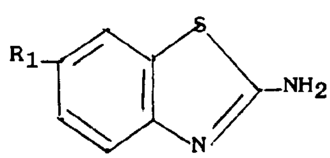

(II)

dans laquelle $R_1$ a les mêmes significations que dans la formule (I) sur un dérivé de formule :

$$R_2 - X \qquad (III)$$

dans laquelle $R_2$ a les mêmes significations que précédemment et X représente un groupe réactif, isole le produit et le transforme éventuellement en sel d'addition avec un acide minéral ou organique.

B - Pour la préparation des composés de formule (I) pour lesquels $R_2$ représente une chaîne $-CH_2-(CH(R_4))n-R_3$ dans laquelle $R_3$ représente un radical alkylsulfonyle ou alkylsulfinyle, $R_4$ représente un atome d'hydrogéne ou un radical alkyle, n est égal à 0 ou 1 et $R_1$ a les mêmes significations que ci-dessus, on oxyde les dérivés correspondants pour lesquels $R_3$ représente un radical alkylthio, isole le produit et le transforme éventuellement en sel d'addition avec un acide minéral ou organique.

C - Pour la préparation des composés de formule (I) pour lesquels $R_2$ représente une chaîne -

$CH_2$-$(CH(R_4))n$-$R_3$ dans laquelle $R_3$ représente un radical mercapto, $R_4$ représente un atome d'hydrogène ou un radical alkyle, n est égal à 0 ou 1 et $R_1$ a les mêmes significations que ci-dessus, on hydrolyse un dérivé correspondant pour lequel $R_3$ représente un radical tert-butylthio, isole le produit et le transforme éventuellement en sel d'addition avec un acide minéral ou organique.

D - Pour la préparation des composés de formule (I) pour lesquels $R_2$ représente un reste de formule (A) et $R_1$ a les mêmes significations que ci-dessus, on fait réagir de l'acide bromhydrique sur une (tert-butylthio-2 éthyl)-3 imino-2 polyfluoroalcoxy ou polyfluoroalkyle-6 benzothiazoline, isole le produit et le transforme éventuellement en sel d'addition avec un acide minéral ou organique.

E - Pour la préparation des composés de formule (I) pour lesquels $R_2$ représente une chaîne -$CH_2$-$(CH(R_4))n$-$R_3$ dans laquelle $R_3$ représente un radical acylthio, on hydrolyse un dérivé de formule :

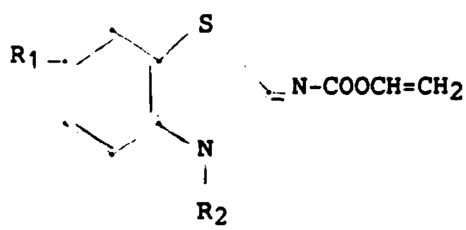

(IV)

dans laquelle $R_1$ a les mêmes significations que ci-dessus, isole le produit et le transforme éventuellement en sel d'addition avec un acide minéral ou organique.

2. Procédé selon la revendication 1 pour la préparation de l'(éthylsulfonyl-2 éthyl)-3 imino-2 trifluorométhoxy-6 benzothiazoline.

3. Procédé selon la revendication 1 Pour la préparation de l'imino-2 (méthylsulfinyl-2 éthyl)-3 trifluorométhoxy-6 benzothiazoline.

## Patentansprüche

### Patentansprüche für folgende Vertragsstaaten : AT, BE, CH, LI, DE, FR, GB, IT, LU, NL, SE

1. Verbindungen der Formel

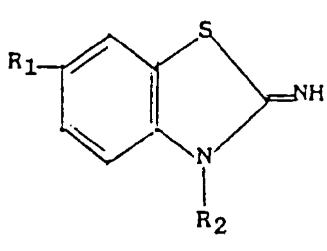

(I)

worin

- $R_1$ einen Polyfluoralkoxy- oder Polyfluoralkylrest bedeutet und
- $R_2$ bedeutet
  . entweder eine Kette -$CH_2$-$(CH(R_4))n$-$R_3$, worin $R_3$ einen Dialkylamino-, Piperidino-, Pyrrolidinyl-1-, Mercapto-, Acylthio-, Alkylthio-, Alkylsulfinyl- oder Alkylsulfonylrest bedeutet, $R_4$ ein Wasserstoffatom oder einen Alkylrest darstellt, und n gleich 0 oder 1 ist,
  . oder einen Rest der Formel

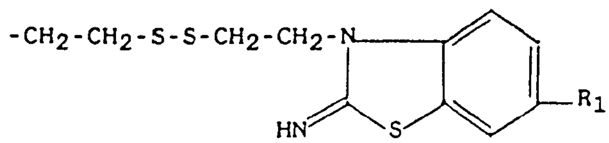

(A)

mit Ausnahme der Verbindungen, für die n gleich 1 ist, $R_3$ einen Dialkylaminorest bedeutet, und $R_4$ einen Methylrest darstellt, wobei die Alkylreste und die Alkyl- und Alkoxyteile 1 bis 4 Kohlenstoffatome in gerader oder verzweigter Kette enthalten und die Acylteile 2 bis 4 Kohlenstoffatome enthalten, sowie die Enantiomeren der Verbindungen, die ein asymmetrisches Zentrum enthalten und die Salze dieser Verbindungen mit einer Mineral- oder organischen Säure.

2. Verfahren zur Herstellung der Verbindungen nach Formel (I) gemäß Anspruch 1 mit Ausnahme solcher, für die $R_2$ einen Rest der Formel (A) darstellt, oder $R_2$ eine Kette $-CH_2-(CH(R_4))n-R_3$ bedeutet, worin $R_3$ einen Alkylthiorest darstellt, dadurch gekennzeichnet, daß man ein Aminderivat der Formel

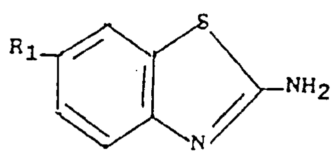

(II)

worin $R_1$ die gleichen Bedeutungen wie in Formel (I) hat, auf ein Derivat der Formel

$R_2$ - X (III)

einwirken läßt, worin $R_2$ die gleichen Bedeutungen wie vorstehend hat, und X eine reaktionsfähige Gruppe darstellt, oder ein Additionssalz einer solchen Verbindung mit einer Mineral- oder organischen Säure, das Produkt isoliert und es gegebenenfalls in ein Additionssalz mit einer Mineral- oder organischen Säure überführt.

3. Verfahren zur Herstellung der Verbindungen der Formel (I) gemäß Anspruch 1, für die $R_2$ eine Kette $-CH_2(CH(R_4))n-R_3$ bedeutet, worin $R_3$ einen Alkylsulfonyl- oder Alkylsulfinylrest darstellt, $R_4$ ein Wasserstoffatom oder einen Alkylrest bedeutet, n gleich 0 oder 1 ist, und $R_1$ die gleichen Bedeutungen wie in Anspruch 1 hat, dadurch gekennzeichnet, daß man die entsprechenden Derivate, für die $R_3$ einen Alkylthiorest bedeutet, oxidiert, das Produkt isoliert, und es gegebenenfalls in ein Additionssalz mit einer Mineral- oder organischen Säure überführt.

4. Verfahren zur Herstellung der Verbindungen der Formel (I) gemäß Anspruch 1, für die $R_2$ eine Kette $-CH_2-(CH(R_4))n-R_3$ bedeutet, worin $R_3$ einen Mercaptorest darstellt, $R_4$ ein Wasserstoffatom oder einen Alkylrest bedeutet, n gleich 0 oder 1 ist, und $R_1$ die gleichen Bedeutungen wie in Anspruch 1 hat, dadurch gekennzeichnet, daß man ein entsprechendes Derivat, worin $R_3$ einen tert.-Butylthiorest darstellt, hydrolysiert, das Produkt isoliert und es gegebenenfalls in ein Additionssalz mit einer Mineral- oder organischen Säure überführt.

5. Verfahren zur Herstellung der Verbindungen der Formel (I) gemäß Anspruch 1, für die $R_2$ einen Rest der Formel (A) darstellt, und $R_1$ die gleichen Bedeutungen wie in Anspruch 1 hat, dadurch gekennzeichnet, daß man Bromwasserstoffsäure auf ein 3-(2-tert.-Butylthio-ethyl)-2-imino-6-polyfluoroalkoxy- oder -polyfluoroalkyl-benzothiazolin einwirken läßt, das Produkt isoliert und es gegebenenfalls in ein Additionssalz mit einer Mineral- oder organischen Säure überführt.

6. Verfahren zur Herstellung der Verbindungen der Formel (I) gemäß Anspruch 1, für die $R_2$ eine Kette $-CH_2-(CH(R_4))n-R_3$ bedeutet, worin $R_3$ einen Alkylthiorest darstellt, dadurch gekennzeichnet, daß man ein Derivat der Formel

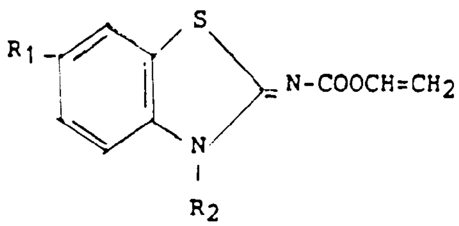

(IV)

hydrolysiert, worin $R_1$ die gleichen Bedeutungen wie in Anspruch 1 hat, und $R_2$ die gleichen Bedeutungen wie vorstehend hat, das Produkt isoliert und es gegebenenfalls in ein Additionssalz mit einer Mineral- oder organischen Säure überführt.

7. Arzneimittel, dadurch gekennzeichnet, daß sie als Wirkstoff mindestens eine Verbindung gemäß Anspruch 1 oder ein Salz einer solchen Verbindung enthalten.

8. Arzneimittel gemäß Anspruch 6 zur Behandlung von Erkrankungen, worin das Glutamat miteinbezogen ist.

9. Verbindung gemäß Anspruch 1: 3-(2-Ethylsulfonyl-ethyl)-2-imino-6-trifluoromethoxy-benzothiazolin.

10. Verbindung gemäß Anspruch 1: 2-Imino-3-(2-methylsulfinyl-ethyl)-6-trifluoromethoxy-benzothiazolin.

**Patentansprüche für folgende Vertragsstaaten : ES, GR**

1. Verfahren zur Herstellung von Verbindungen der Formel

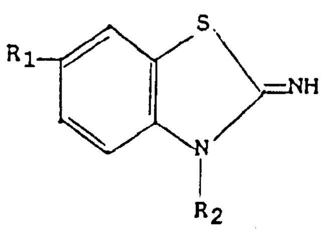

(I)

worin
- $R_1$ einen Polyfluoralkoxy- oder Polyfluoralkylrest bedeutet und
- $R_2$ bedeutet
  . entweder eine Kette $-CH_2-(CH(R_4))n-R_3$, worin $R_3$ einen Dialkylamino-, Piperidino-, Pyrrolidinyl-1-, Mercapto-, Acylthio-, Alkylthio-, Alkylsulfinyl- oder Alkylsulfonylrest bedeutet, $R_4$ ein Wasserstoffatom oder einen Alkylrest darstellt, und n gleich 0 oder 1 ist,
  . oder einen Rest der Formel

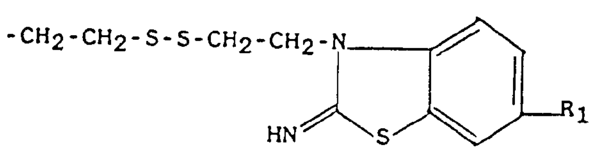

(A)

mit Ausnahme der Verbindungen, für die n gleich 1 ist, $R_3$ einen Dialkylaminorest bedeutet, und $R_4$ einen Methylrest darstellt, wobei die Alkylreste und die Alkyl- und Alkoxyteile 1 bis 4 Kohlenstoffatome in gerader oder verzweigter Kette enthalten und die Acylteile 2 bis 4 Kohlenstoffatome enthalten, sowie die Enantiomeren der Verbindungen, die ein asymmetrisches Zentrum enthalten, und die Salze dieser Verbindungen mit einer Mineral- oder organischen Säure, dadurch gekennzeichnet, daß man:

A - zur Herstellung von Verbindungen der Formel (I) mit Ausnahme solcher, für die $R_2$ einen Rest der Formel (A) darstellt, oder $R_2$ eine Kette $-CH_2(CH(R_4))n-R_3$ bedeutet, worin $R_3$ einen Acylthio-

rest darstellt, ein Aminderivat der Formel

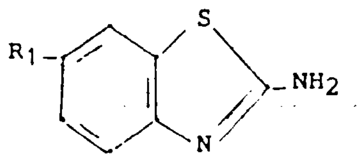

(II)

worin $R_1$ die gleichen Bedeutungen wie in Formel (I) hat, auf ein Derivat der Formel

$R_2$ - X (III)

einwirken läßt, worin $R_2$ die gleichen Bedeutungen wie vorstehend hat, und X eine reaktionsfähige Gruppe darstellt, das Produkt isoliert und es gegebenenfalls in ein Additionssalz mit einer Mineral- oder organischen Säure überführt.

B - zur Herstellung von Verbindungen der Formel (I), für die $R_2$ eine Kette $-CH_2-(CH(R_4))nR_3$ bedeutet, worin $R_3$ einen Alkylsulfonyl- oder Alkylsulfinylrest darstellt, $R_4$ ein Wasserstoffatom oder einen Alkylrest bedeutet, n gleich 0 oder 1 ist, und $R_1$ die gleichen Bedeutungen wie vorstehend hat, die entsprechenden Derivate, für die $R_3$ einen Alkylthiorest darstellt, oxidiert, das Produkt isoliert, und es gegebenenfalls in ein Additionssalz mit einer Mineral- oder organischen Säure überführt;

C - zur Herstellung von Verbindungen der Formel (I), für die $R_2$ eine Kette $-CH_2-(CH(R_4))n-R_3$ bedeutet, worin $R_3$ einen Mercaptorest darstellt, $R_4$ ein Wasserstoffatom oder einen Alkylrest bedeutet, n gleich 0 oder 1 ist, und $R_1$ die gleichen Bedeutungen wie vorstehend hat, ein entsprechendes Derivat, worin $R_3$ einen tert.-Butylthiorest darstellt, hydrolysiert, das Produkt isoliert und es gegebenenfalls in ein Additionssalz mit einer Mineral- oder organischen Säure überführt;

D - zur Herstellung der Verbindungen der Formel (I), für die $R_2$ einen Rest der Formel (A) darstellt, und $R_1$ die gleichen Bedeutungen wie in Anspruch 1 hat, Bromwasserstoffsäure auf ein 3-(2-tert.-Butylthio-ethyl)-2-imino-6-polyfluoroalkoxy- oder -polyfluoroalkyl-benzothiazolin einwirken läßt, das Produkt isoliert und es gegebenenfalls in ein Additionssalz mit einer Mineral- oder organischen Säure überführt;

E - zur Herstellung von Verbindungen der Formel (I), für die $R_2$ eine Kette $-CH_2-(CH(R_4))n-R_3$ bedeutet, worin $R_3$ einen Acylthiorest darstellt, ein Derivat der Formel

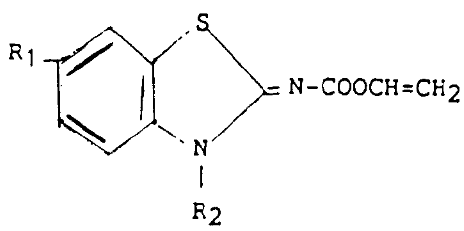

(IV)

worin $R_1$ die gleichen Bedeutungen wie vorstehend hat, hydrolysiert das Produkt isoliert und es gegebenenfalls in ein Additionssalz mit einer Mineral- oder organischen Säure überführt.

2. Verfahren gemäß Anspruch 1 zur Herstellung von 3-(2-Ethylsulfonyl-ethyl)-2-imino-6-trifluoromethoxy-benzothiazolin.

3. Verfahren gemäß Anspruch 1 zur Herstellung von 2-Imino-3-(2-methylsulfinylethyl)-6-trifluoromethoxy-benzothiazolin.

## Claims

**Claims for the following Contracting States : AT, BE, CH, LI, DE, FR, GB, IT, LU, NL, SE**

1. Compounds of formula:

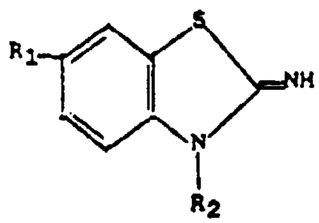

(I)

in which

- $R_1$ denotes a polyfluoroalkoxy or polyfluoroalkyl radical, and
- $R_2$ denotes
  . either a chain $-CH_2-(CH(R_4))n-R_3$, in which $R_3$ denotes a dialkylamino, piperidino, 1-pyrrolidinyl, mercapto, acylthio, alkylthio, alkylsulphinyl or alkylsulphonyl radical, $R_4$ denotes a hydrogen atom or an alkyl radical and n is equal to 0 or 1,
  . or a residue of formula:

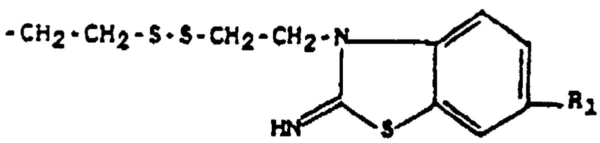

(A)

with the exception of the compounds for which n is equal to 1, $R_3$ denotes a dialkylamino radical and $R_4$ denotes a methyl radical, on the understanding that the alkyl radicals and alkyl and alkoxy portions contain 1 to 4 carbon atoms in a straight or branched chain and the acyl portions contain 2 to 4 carbon atoms, as well as the enantiomers of the compounds containing an asymmetric centre and the salts of these compounds with an inorganic or organic acid.

**2.** A process for preparing the compounds of formula (I) according to claim 1, with the exception of those for which $R_2$ denotes a residue of formula (A) or $R_2$ denotes a chain $-CH_2-(CH(R_4))n-R_3$ in which $R_3$ denotes an acylthio radical, wherein an amino derivative of formula:

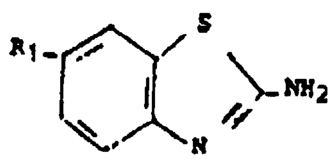

(II)

in which $R_1$ has the same meanings as in the formula (I), is reacted with a derivative of formula:

$$R_2 - X \qquad (III)$$

in which $R_2$ has the same meanings as above and X denotes a reactive group, or an addition salt of such a compound with an inorganic or organic acid, and the product is isolated and optionally converted to an addition salt with an inorganic or organic acid.

**3.** A process for preparing the compounds of formula (I) according to claim 1 for which $R_2$ denotes a chain $-CH_2-(CH(R_4))n-R_3$ in which $R_3$ denotes an alkylsulphonyl or alkylsulphinyl radical, $R_4$ denotes a hydrogen atom or an alkyl radical, n is equal to 0 or 1 and $R_1$ has the same meanings as in claim 1, wherein the corresponding derivatives for which $R_3$ denotes an alkylthio radical are oxidised, and the product is isolated and optionally converted to an addition salt with an inorganic or organic acid.

**4.** A process for preparing the compounds of formula (I) according to claim 1 for which $R_2$ denotes a chain $-CH_2-(CH(R_4))n-R_3$ in which a denotes a mercapto radical, $R_4$ denotes a hydrogen atom or an alkyl radical, n is equal to 0 or 1 and $R_1$ has the same meanings as in claim 1, wherein a corresponding derivative for which $R_3$ denotes a tert-butylthio radical is hydrolysed, and the product is isolated and optionally converted to an addition salt with an inorganic or organic acid.

5. A process for preparing the compounds of formula (I) according to claim 1 for which $R_2$ denotes a residue of formula (A) and $R_1$ has the same meanings as in claim 1, wherein hydrobromic acid is reacted with a 3-(2-tert-butylthioethyl)-2-imino-6-(polyfluoroalkoxy)- or -(polyfluoroalkyl)benzothiazoline, and the product is isolated and optionally converted to an addition salt with an inorganic or organic acid.

6. A process for preparing the compounds of formula (I) according to claim 1 for which $R_2$ denotes a chain $-CH_2-(CH(R_4))n-R_3$ in which $R_3$ denotes an acylthio radical, wherein a derivative of formula:

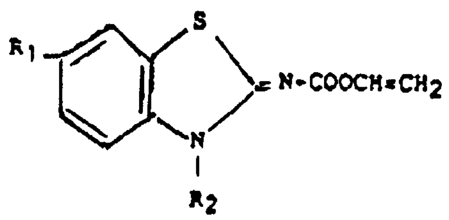

(IV)

in which $R_1$ has the same meanings as in claim 1 and $R_2$ has the same meanings as above, is hydrolysed, and the product is isolated and optionally converted to an addition salt with an inorganic or organic acid.

7. Medicinal products, which contain, as active principle, at least one compound according to claim 1, or a salt of such a compound.

8. Medicinal products according to claim 6, for the treatment of conditions in which glutamate is implicated.

9. Compound according to claim 1: 3-(2-Ethylsulphonylethyl)-2-imino-6-(trifluoromethoxy)benzothiazoline.

10. Compound according to claim 1: 2-Imino-3-(2-methylsulphinylethyl)-6-(trifluoromethoxy)benzothiazoline.

**Claims for the following Contracting States : ES, GR**

1. A process for preparing the compounds of formula:

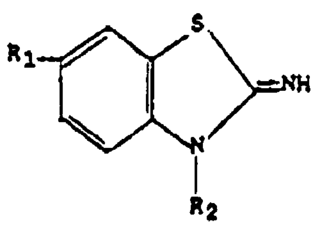

(I)

in which

- $R_1$ denotes a polyfluoroalkoxy or polyfluoroalkyl radical, and
- $R_2$ denotes
  . either a chain $-CH_2-(CH(R_4))n-R_3$, in which $R_3$ denotes a dialkylamino, piperidino, 1-pyrrolidinyl, mercapto, acylthio, alkylthio, alkylsulphinyl or alkylsulphonyl radical, $R_4$ denotes a hydrogen atom or an alkyl radical and n is equal to 0 or 1,
  . or a residue of formula:

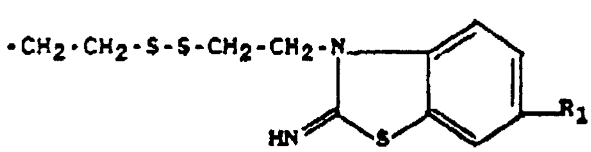

(A)

with the exception of the compounds for which n is equal to 1, $R_3$ denotes a dialkylamino radical and $R_4$ denotes a methyl radical, on the understanding that the alkyl radicals and alkyl and alkoxy

portions contain 1 to 4 carbon atoms in a straight or branched chain and the acyl portions contain 2 to 4 carbon atoms, as well as the enantiomers of the compounds containing an asymmetric centre and the salts of these compounds with an inorganic or organic acid, wherein:

A - for the preparation of the compounds of formula (I), with the exception of those for which $R_2$ denotes a residue of formula (A) or $R_2$ denotes a chain $-CH_2-(CH(R_4))n-R_3$ in which $R_3$ denotes an acylthio radical, an amino derivative of formula:

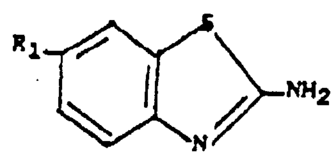

(II)

in which $R_1$ has the same meanings as in the formula (I), is reacted with a derivative of formula:

$R_2$ - X (III)

in which $R_2$ has the same meanings as above and X denotes a reactive group, and the product is isolated and optionally converted to an addition salt with an inorganic or organic acid.

B - For the preparation of the compounds of formula (I) for which $R_2$ denotes a chain $-CH_2-(CH(R_4))n-R_3$ in which $R_3$ denotes an alkylsulphonyl or alkylsulphinyl radical, $R_4$ denotes a hydrogen atom or an alkyl radical, n is equal to 0 or 1 and $R_1$ has the same meanings as above, the corresponding derivatives for which $R_3$ denotes an alkylthio radical are oxidised, and the product is isolated and optionally converted to an addition salt with an inorganic or organic acid.

C - for the preparation of the compounds of formula (I) for which $R_2$ denotes a chain $-CH_2-(CH(R_4))n-R_3$, in which $R_3$ denotes a mercapto radical, $R_4$ denotes a hydrogen atom or an alkyl radical, n is equal to 0 or 1 and $R_1$ has the same meanings as above, a corresponding derivative for which $R_3$ denotes a tert-butylthio radical is hydrolysed, and the product is isolated and optionally converted to an addition salt with an inorganic or organic acid.

D - for the preparation of the compounds of formula (I) for which $R_2$ denotes a residue of formula (A) and $R_1$ has the same meanings as above, hydrobromic acid is reacted with a 3-(2-tert-butylthioetnyl)-2-imino-6-(polyfluoroalkoxy)- or -(polyfluoroalkyl)benzothiazoline, and the product is isolated and optionally converted to an addition salt with an inorganic or organic acid.

E - For the preparation of the compounds of formula (I) for which $R_2$ denotes a chain $-CH_2-(CH(R_4))n-R_3$ in which $R_3$ denotes an acylthio radical, a derivative of formula:

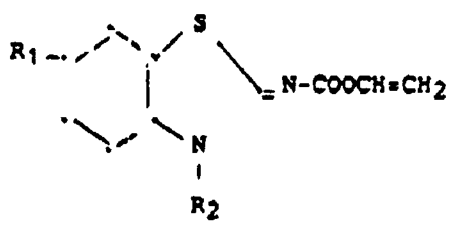

(IV)

in which $R_1$ has the same meanings as above, is hydrolysed, and the product is isolated and optionally converted to an addition salt with an inorganic or organic acid.

2. The process according to claim 1, for the preparation of 3-(2-ethylsulphonylethyl)-2-imino-6-(trifluoromethoxy)benzothiazoline.

3. The process according to claim 1, for the preparation of 2-imino-3-(2-methylsulphinylethyl)-6-(trifluoromethoxy)benzothiazoline.